# FASCICULE DE BREVET EUROPEEN

(11) **EP 3 569 220 B1**
(45) Date de publication et mention de la délivrance du brevet: **28.04.2021**
(21) Numéro de dépôt: 19174174.3
(22) Date de dépôt: 13.05.2019
(51) Int. Cl.: A61K 8/34, A61K 8/49, A61Q 19/00, C11D 1/00

(54) **COMPOSITION TENSIOACTIVE A BASE D'ESTERS D'OLIGOMERES D'ISOSORBIDE ET DE 1,3-PROPANEDIOL**
TENSIDE AUF DER BASIS VON OLIGOMERESTERN VON ISOSORBID UND 1,3-PROPANDIOL
SURFACTANT COMPOSITION MADE OF ESTERS OF ISOSORBIDE- AND 1.3-PROPANEDIOL-OLIGOMERS

(30) Priorité: 16.05.2018 FR 1854080
(43) Date de publication de la demande: 20.11.2019
(73) Titulaire: GATTEFOSSE SAS, 69804 Saint Priest (FR)
(72) Inventeur: RODIER, Jean-David, 69100 VILLEURBANNE (FR)
(74) Mandataire: Cabinet Laurent & Charras

(56) Documents cités:
- FR-A1- 2 980 694
- RODIER,RITTER,DALICIEUX,SAUTET: "Isosorbide telechelic bio-based oligomers", JOURNAL OF POLYMER SCIENCE, vol. 55, 2 mai 2017 (2017-05-02), pages 2178-2189, XP002789506,

## Description

La présente invention est relative au domaine des tensioactifs, en particulier les tensioactifs utilisés dans des compositions cosmétiques.

L'invention concerne plus particulièrement un tensioactif à base d'oligoéthers d'isosorbide et de 1,3-propanediol estérifiés avec un acide gras.

Le développement de produits issus de matières premières biosourcées constitue un enjeu majeur dans le but de proposer une alternative à l'exploitation des matières premières d'origine fossile.

A titre d'exemple, le 1,3-propanediol, qui peut être obtenu à partir de glycérol ou de sirop de glucose provenant du maïs, est utilisé pour préparer des polyuréthanes ou des polyesters. En particulier, l'homopolymère du 1,3-propanediol, ou PTEG pour polytriméthylène éther glycol, est commercialisé sous le nom Cerenol® par la société Dupont.

La solubilité du PTEG dans l'eau diminuant avec la masse molaire, sa structure a été modifiée afin d'éviter l'apparition de deux phases à température ambiante. A titre d'exemple, des oligoéthers issus de l'association entre les monomères 1,3-propanediol (PDO) et isosorbide (ISO) ont été synthétisés et comparés à l'homopolymère de PDO, c'est-à-dire au PTEG (Rodier et al., « Isosorbide Telechelic Bio-Based Oligomers », Journal of Polymer Science, Part A : Polymer Chemistry, 2017, 55, 2178-2189). Il a ainsi été observé que l'incorporation d'unités isosorbide dans le PTEG permettait d'augmenter la solubilité dans l'eau des oligoéthers, et ce, à masse molaire équivalente. Ces travaux ont donc mis en évidence que la faible hydrosolubilité du PTEG pouvait être modifiée en copolymérisant le 1,3-propanediol et l'isosorbide, ce qui permet également de résoudre le problème d'apparition de deux phases à température ambiante.

Cependant, la copolymérisation du 1,3-propanediol et de l'isosorbide génère un mélange de produits comprenant de l'isosorbide n'ayant pas copolymérisé et ne pouvant pas être éliminé aisément.

Un tensioactif comprend une partie hydrophobe et une partie hydrophile. Ainsi, la fonctionnalisation de l'isosorbide avec un acide gras conduit à un tensioactif puisque l'isosorbide est un composé hydrophile. Cela peut paraître surprenant dans la mesure où les constituants hydrophiles des tensioactifs utilisés pour préparer une émulsion huile dans eau stable présentent généralement de plus hautes masses molaires que l'isosorbide.

Le Demandeur a constaté, de manière tout à fait inattendue, que l'élimination de l'isosorbide libre avant de réaliser le greffage permettait d'atteindre les performances souhaitées, et que cette élimination pouvait être résolue grâce à la technique de distillation moléculaire.

Ainsi la présente invention concerne une composition tensioactive à base d'au moins un ester d'oligomères d'isosorbide et de 1,3-propanediol.

Plus précisément, l'invention concerne une composition tensioactive comprenant un mélange M1 constitué d'oligomères de formule (I) :

R1-O-(ISO-PDO_{X})_{N}-ISO_{X'}-OR2 (I)

- -O-(ISO-PDO_{X})_{N}-ISO_{X'}O- représentant un oligoéther comprenant au moins un résidu d'un monomère 1,3-propanediol (PDO) et au moins un résidu d'un monomère isosorbide (ISO),
- R1 et R2 étant un atome d'hydrogène ou un groupement R3C(=O)-,
- R1 et R2 étant identiques ou différents,
- R3 étant une chaine carbonée comprenant entre 7 et 21 atomes de carbone, saturée ou insaturée, linéaire ou ramifiée,
chaque oligomère de formule (I) présentant un rapport molaire ISO/PDO compris entre 20/80 et 50/50 et une masse molaire inférieure ou égale à 1500 g.mol⁻¹,
le mélange M1 comprenant au moins 25 % en masse, avantageusement au moins 50 % en masse, d'au moins un oligomère de formule (I) dont au moins un des groupes R1 ou R2 est un groupement R3C(=O)-,
la composition comprenant, par rapport à la masse du mélange M1, moins de 10% en masse de composé de formule (II) :

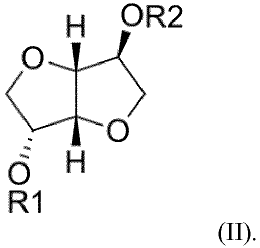

La masse molaire est la masse molaire moyenne en nombre, Mₙ, des oligomères.

En fonction de la quantité des monomères ISO et PDO et des acides gras, il est possible de moduler les propriétés hydrophiles et hydrophobes de la composition tensioactive selon l'invention.

De manière générale, la composition tensioactive comprend moins de 10 % en masse de composé de formule (II).

Dans la formule (I), x est un nombre moyen compris entre 1 et 5, avantageusement entre 1 et 3. D'autre part, x' est un nombre moyen compris entre 0 et 1, avantageusement entre 0,5 et 1. N est un nombre moyen entre 1 et 5, avantageusement entre 1 et 3.

Par « nombre moyen », on désigne le nombre moyen de motifs ISO et/ou PDO des composés de formule (I) du mélange M1.

La composition tensioactive selon l'invention présente des propriétés tensioactives grâce au mélange M1 dont les oligomères ayant R1 et/ou R2 = R3C(=O)- comme partie lipophile et une partie hydrophile (oligoéther) O-(ISO-PDO_{X})_{N}-ISO_{X'}-O-.

Ces propriétés tensioactives permettent de modifier la tension interfaciale entre deux milieux, par exemple entre un milieu aqueux et un milieu organique (huile...).

Il s'agit avantageusement d'une composition tensioactive de type eau dans huile.

### Oligoéther -O-(ISO-PDO_{X})_{N}-ISO_{X'}-O-

Conformément à l'invention, -O-(ISO-PDO_{X})_{N}-ISO_{X'}-O- représente un oligoéther comprenant au moins une unité monomère PDO et au moins une unité monomère ISO.

Ainsi, -O-(ISO-PDO_{X})_{N}-ISO_{X'}-O- représente une succession d'unités de monomères isosorbide et 1,3-propanediol liés entre eux par des liaisons éther où les unités ISO sont séparés par au moins une unité PDO. Il a été démontré que la succession ISO-ISO n'était pas observée (Rodier et al., « Isosorbide Telechelic Bio-Based Oligomers », Journal of Polymer Science, Part A : Polymer Chemistry, 2017, 55, 2178-2189). Néanmoins, de manière avantageuse, l'oligoéther -O-(ISO-PDO_{X})_{N}-ISO_{X'}-O- comprend au moins un bloc PDOx alterné par un motif ISO.

On entend par succession d'unités de monomères isosorbide et 1,3-propanediol liés entre eux par des liaisons éthers une chaine carbonée polyéther pouvant correspondre à la formule (III) : 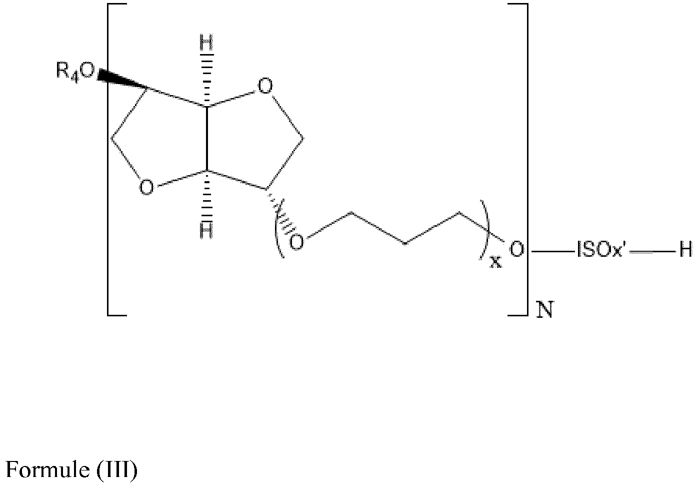 dans laquelle :
x est un nombre moyen compris entre 1 et 5, avantageusement entre 1 et 3,
x' est un nombre moyen compris entre 0 et 1, avantageusement entre 0,5 et 1,
N est un nombre moyen entre 1 et 5, avantageusement entre 1 et 3, et
R4 = H ou PDOₓ.

Bien entendu, x, x' et N correspondent aux nombres moyens de motifs ISO et/ou PDO des composés de formule (III) du mélange M1.

La formule (III) représente la chaine -O-(ISO-PDO_{X})_{N}-ISO_{X'}-O- qui correspond à un mode de réalisation particulier de la formule (I).

Bien entendu, la formule (III) ci-dessus n'est donnée qu'à titre d'illustration et l'homme du métier comprend que le nombre d'unités de monomères 1,3-propanediol entre deux unités monomères isosorbide n'est pas systématiquement identique au sein d'un même oligomère. Cependant, le Demandeur a remarqué que, de manière générale et certainement pour des raisons de réactivité liée à l'encombrement stérique, les oligomères de la composition selon l'invention ne comprennent pas deux unités consécutives de monomères d'isosorbide.

Le rapport molaire ISO/PDO entre les unités de monomères isosorbide et 1,3-propanediol dans les oligomères de formule (I) est compris entre 20/80 et 50/50, avantageusement entre 30/70 et 50/50, et encore plus avantageusement entre 30/70 et 40/60.

De manière avantageuse, ce rapport molaire correspond également au rapport entre toutes les unités ISO et toutes les unités PDO de tous les oligomères de formule (I) du mélange M1. Il s'agit alors du rapport molaire global ISO/PDO du mélange M1.

L'oligoéther -O-(ISO-PDO_{X})_{N}-ISO_{X'}-O- peut se terminer par deux unités isosorbide, ou par une unité isosorbide ISO et une unité 1,3-propanediol ou par deux unités 1,3-propanediol. De manière avantageuse, l'oligoéther -O-(ISO-PDO_{X})_{N}-ISO_{X'}-O- se termine par au moins une unité isosorbide (résidu de monomère d'isosorbide), avantageusement par deux unités de monomère ISO.

De manière avantageuse, 10 à 100% des oligoéthers -O-(ISO-PDO_{X})_{N}-ISO_{X'}-Odes oligomères de formule (I) du mélange M1 présentent au moins un motif terminal qui est une unité isosorbide ISO (résidu de monomère d'isosorbide), plus avantageusement 50 à 100%, et encore plus avantageusement 70 à 100 %.

De manière avantageuse, 10 à 100% des oligoéthers -O-(ISO-PDO_{X})_{N}-ISO_{X'}-Odes oligomères de formule (I) du mélange M1 présentent deux motifs terminaux qui sont des unités isosorbide ISO, plus avantageusement 50 à 100%, et encore plus avantageusement 70 à 100 %.

### Oligomère de formule (I)

Conformément à l'invention, au moins un oligomère de formule (I) comprend au moins un des groupes R1 ou R2 où R1 ou R2 est un groupe R3C(=O)-, R3 étant une chaine carbonée comprenant entre 7 et 21 atomes de carbone, saturée ou insaturée, linéaire ou ramifiée. La chaîne carbonée R3 peut éventuellement contenir un ou plusieurs hétéroatomes, par exemple l'oxygène et/ou l'azote.

Selon un mode de réalisation particulier de l'invention, le mélange M1 peut comprendre les trois types d'oligomères suivants :
- des oligomères de formule (I) avec R1 et R2 étant simultanément des groupes R3C(=O)- identiques ou non, et
- des oligomères de formule (I) avec R1 étant un groupe R3C(=O)-, R2 étant un atomes d'hydrogène, et
- des oligomères de formule (I) avec R1 et R2 étant simultanément des atomes d'hydrogène.

Avantageusement, au moins 40% des groupes R1 et R2 des oligomères de formule (I) du mélange M1 sont des groupes R3C(=O)-, plus avantageusement entre 40 et 70 %, et encore plus avantageusement entre 50 et 60%.

Ainsi, selon un mode de réalisation particulier, la composition selon l'invention peut comprendre :
- 5 à 80% d'oligomères R3C(=O)-O-(ISO-PDO_{X})_{N}-ISO_{X'}-O-C(=O)R3, plus avantageusement 5 à 30% (R1 = R2 = C(=O)R3),
- 10 à 70% ou 20 à 70% d'oligomères R3C(=O)-O-(ISO-PDO_{X})_{N}-ISO_{X'}-OH, plus avantageusement 30 à 60% (R1 = C(=O)R3 et R2 = H),
- 0 à 40 % d'oligoéthers HO-(ISO-PDO_{X})_{N}-ISO_{X'}-OH, plus avantageusement 0 à 30 %(R1 = R2 = H).
   les pourcentages étant exprimés en masse, par rapport à la masse de la composition tensioactive.

Comme déjà indiqué, R3 est une chaine carbonée comprenant 7 à 21 atomes de carbone.

De manière avantageuse, R3 est une chaine linéaire ou ramifiée, saturée ou insaturée. De manière encore plus avantageuse, R3 est une chaine alkyle.

R3 est avantageusement une chaine composée exclusivement d'atomes de carbone et d'atomes d'hydrogène, comprenant avantageusement entre 9 et 19 atomes de carbone, plus avantageusement entre 11 et 17 atomes de carbone, et encore plus avantageusement entre 13 et 17 atomes de carbone.

Ainsi, R3 représente avantageusement un groupe alkyl linéaire de formule CₙH₂ₙ₊₁, n étant un nombre entier de 7 à 21, avantageusement de 9 à 19, plus avantageusement de 11 à 17, et encore plus avantageusement de 13 à 17.

Avantageusement, le motif R3C(=O)- est issu de l'estérification d'un acide gras avec une fonction OH terminale de l'oligoéther HO-(ISO-PDO_{X})_{N}-ISO_{X'}-OH

De manière avantageuse, l'acide gras est choisi dans le groupe comprenant l'acide stéarique et l'acide palmitique.

Conformément à l'invention, la masse molaire moyenne des oligomères de formule (I) est inférieure ou égale à 1500 g.mol¹ et supérieure à 400 g/mol. De manière avantageuse, elle est inférieure ou égale à 1000 g.mol_{"}¹. De manière générale, la masse molaire moyenne des oligomères est au moins égale à 400 g/mol.

Le composé de formule (II) correspond à l'isosorbide n'ayant pas réagi (R1 = R2 = H) lors de la synthèse de l'oligoéther) HO-(ISO-PDO_{X})_{N}-ISO_{X'}-OH et/ou à son dérivé (R1 et/ou R2 = R3C(=O)-) produit lors de la formation des oligomères de formule (I). Le dérivé (R1 et/ou R2 = R3C(=O)-) peut donc correspondre au produit d'estérification entre l'isosorbide et un acide gras.

De manière avantageuse, la composition selon l'invention comprend moins de 10% en masse de composé de formule (II), plus avantageusement moins de 5%, et encore plus avantageusement moins de 3%.

Idéalement, la composition selon l'invention comprend 0 % en masse de composé de formule (II), dans la limite des techniques conventionnelles de détection.

### Procédé

L'invention concerne également un procédé de préparation de ladite composition comprenant le mélange M1 d'oligomères de formule (I).

Ce procédé de préparation d'une composition comprenant un mélange M1 d'oligomères de formule (I) comprend au moins les étapes suivantes :
a) préparation d'une composition A comprenant un mélange d'oligoéthers par polyétherification de 1,3-propanediol (PDO) et d'isosorbide (ISO), le rapport molaire initial ISO/PDO étant compris entre 40/60 et 70/30, avantageusement entre 45/55 et 55/45,
b) purification, par distillation moléculaire, de la composition A pour obtenir une composition B comprenant moins de 4% en masse d'isosorbide n'ayant pas été polyétherifié, par rapport à la masse de la composition B,
c) estérification, avantageusement par voie enzymatique, des oligoéthers de la composition B avec au moins un acide carboxylique de formule R3C(=O)OH, R3 étant une chaine carbonée comprenant entre 7 et 21 atomes de carbone, saturée ou insaturée, linéaire ou ramifiée.

Les caractéristiques de la composition, du mélange M1, des oligoéthers, des oligomères de formule (I) et du groupement R3 sont tels qu'indiquées dans la description ci-avant.

L'étape a) de polyétherification est avantageusement réalisée selon la méthode décrite dans l'article « Isosorbide Telechelic Bio-Based Oligomers » (Rodier et al., Journal of Polymer science, Part A : Polymer Chemistry 2017, 55, 2178-2189).

Le produit issu de l'étape a) contient avantageusement moins de 30% d'isosorbide libre en masse. La teneur en isosorbide libre est généralement déterminée par chromatographie en phase gazeuse. La masse molaire moyenne de l'oligoéther déterminée par résonance magnétique nucléaire (RMN) du proton (¹H RMN), et sans prendre en compte l'isosorbide libre, peut être comprise entre 300 et 1000 g/mol.

L'étape b) de purification permet d'éliminer au moins en partie l'isosorbide n'ayant pas réagi lors de l'étape a) de polyétherification. Elle est réalisée par distillation moléculaire.

L'équipement utilisé peut être un distillateur moléculaire de modèle KDL 5 (UIC Gmbh). A titre d'exemple, la température de la double enveloppe du distillateur moléculaire peut être fixée à 130°C, celle du condenseur interne à 70°C, soit 8°C au-dessus de la température de fusion d'isosorbide, la vitesse d'agitation à 300 tours/min. La pression de travail peut alors être de l'ordre de 6.10⁻² mbar soit 6 Pa.

L'étape b) de distillation est avantageusement réalisée à une température comprise entre 110 et 150°C, plus avantageusement entre 120 et 140°C.

La distillation est avantageusement réalisée à une pression comprise entre 0.05 mbar (5 Pa) et 0.1 mbar (10 Pa), plus avantageusement entre 0.05 mbar (5 Pa) et 0.08 mbar (8 Pa) (1 mbar = 100 Pa).

L'élimination de l'isosorbide n'ayant pas réagi lors de l'étape a) permet également de montrer que les propriétés tensioactives de la composition selon l'invention proviennent de la composition B des exemples.

L'étape c) d'estérification est avantageusement une estérification par voie enzymatique. Elle peut donc être avantageusement réalisée en présence d'une enzyme, par exemple une lipase. L'enzyme est avantageusement la lipase *Candida Antarctica B.*

L'enzyme peut se présenter sous la forme d'une enzyme supportée.

L'invention concerne également l'utilisation de ladite composition comprenant un mélange d'oligomères de formule (I) en tant que tensioactif.

L'invention concerne également des formulations cosmétiques contenant ladite composition comprenant un mélange d'oligomères de formule (I) en tant que tensioactif. Ces formulations cosmétiques peuvent être des émulsions, notamment des émulsions eau dans huile.

L'invention et les avantages qui en découlent ressortiront mieux de la figure et exemples suivants donnés afin d'illustrer l'invention et non de manière limitative.

### FIGURE

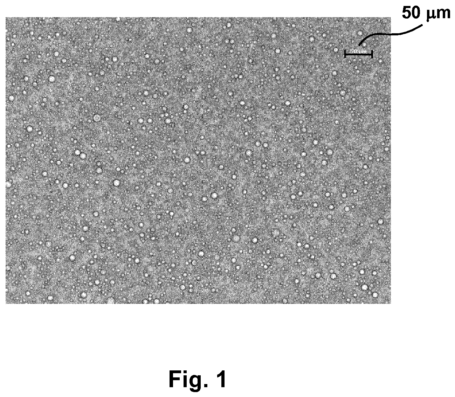

La figure 1 illustre le grossissement x20 (échelle de 50 µm) d'une émulsion huile dans eau obtenue à partir de la composition tensioactive selon l'invention.

### EXEMPLES

Une composition comprenant un mélange d'oligoéthers H-O-(ISO-PDO_{X})_{N}-ISO_{X'}-OH ayant une masse molaire moyenne de 580 g.mol⁻¹ est obtenue en suivant le protocole de l'article « Isosorbide Telechelic Bio-Based Oligomers » (Rodier et al., Journal of Polymer science, Part A : Polymer Chemistry 2017, 55, 2178-2189).

Ce mélange d'oligoéthers contient 26,9 % d'isosorbide libre en masse. La teneur en isosorbide libre a été déterminée par chromatographie en phase gazeuse.

Le mélange d'oligoéthers ainsi obtenu est ensuite purifié, par distillation moléculaire, pour conduire à une composition comprenant des oligoéthers H-O-(ISO-PDO_{X})_{N}-ISO_{X'}-OH. Plus précisément, la quantité d'isosorbide distillée correspond à 25,8% de la masse introduite dans le distillateur. Cette fraction distillée est constituée à 96,1% en masse d'isosorbide. La teneur est déterminée par chromatographie en phase gazeuse. L'oligoéther récupéré ne contient plus que 2,7% d'isosorbide en masse. La masse molaire moyenne de H-O-(ISO-PDO_{X})_{N}-ISO_{X'}-OH, déterminée par RMN ¹H, sans tenir compte de l'isosorbide libre, est de 580 g/mol.

Pour la purification, l'équipement utilisé est un distillateur moléculaire de modèle KDL 5 (UIC Gmbh). La température de la double enveloppe du distillateur moléculaire est fixée à 130°C, celle du condenseur interne à 70°C, la vitesse d'agitation à 300 tours/min. La pression de travail est de 6.10⁻² mbar (0,06 Pa). De l'acide stéarique est ajouté à la composition avec un rapport molaire de 1/1 entre les oligoéthers HO-(ISO-PDO_{X})_{N}-ISO_{X'}-OH et l'acide stéarique (C₁₇H₃₅COOH), puis 5% en masse de la lipase de *Candida Antartica B* supportée est introduite pour catalyser la réaction d'estérification. La réaction est avantageusement conduite à 80°C sous azote, pendant 24 heures.

Une composition comprenant le mélange d'oligomères est analysée par HPLC-DEDL. La composition suivante est déterminée :
- 25 % aire du chromatogramme de HO-(ISO-PDO_{X})_{N}-ISO_{X'}-OH,
- 48 % aire du chromatogramme de monostéarate C₁₇H₃₅C(=O-O-(ISO-PDO_{X})_{N}-ISO_{X'}OH,
- 27 % aire du chromatogramme de distéarate C₁₇H₃₅C(=O)-O-(ISO-PDO_{X})_{N}-ISO_{X'}-O-C(=O)C₁₇H₃₅.

Ce mélange d'oligomères est ensuite utilisé pour former une émulsion.

L'émulsion réalisée est composée en masse de 5% du mélange d'oligomères, 15% d'un mélange huileux comprenant en quantité équivalente du caprylocaprate de glycérol et du polydécène hydrogéné, de l'isostéarate d'isostéaryle et de l'huile végétale, et enfin 80 % d'eau déminéralisée. L'oligomère est introduit dans le mélange huileux puis porté à 75-80°C de même que l'eau. Dès que la température souhaitée est atteinte, la phase huileuse est introduite dans l'eau sous agitation. L'ensemble est ensuite refroidi sous agitation.

Comme le montre la figure 1, l'émulsion huile dans eau ainsi obtenue est fine.

Elle a été conservée plusieurs mois à température ambiante sans qu'aucune dégradation n'ait été constatée.

La même préparation est réalisée à partir de la composition d'oligoéthers B, de la même phase huileuse que précédemment et de l'eau. Une fois la préparation obtenue et refroidie, on observe une séparation de phase. Le mélange d'oligoéthers B n'a pas la capacité à réaliser les émulsions.

Ainsi, la composition selon l'invention permet l'obtention d'une émulsion fine et stable dans le temps.

## Revendications

1. Composition tensioactive comprenant un mélange M1 constitué d'oligomères de formule (I) :
R1-O-(ISO-PDO_{X})_{N}-ISO_{X'}-OR2 (I)
- -O-(ISO-PDO_{X})_{N}-ISO_{X'}-O- représentant un oligoéther comprenant au moins un résidu d'un monomère 1,3-propanediol (PDO) et au moins un résidu d'un monomère isosorbide (ISO),
- R1 et R2 étant un atome d'hydrogène ou un groupement R3C(=O)-,
- R1 et R2 étant identiques ou différents,
- R3 étant une chaine carbonée comprenant entre 7 et 21 atomes de carbone, saturée ou insaturée, linéaire ou ramifiée,
- x étant un nombre moyen compris entre 1 et 5,
- x' étant un nombre moyen compris entre 0 et 1,
- N étant un nombre moyen entre 1 et 5,
chaque oligomère de formule (I) présentant un rapport molaire ISO/PDO compris entre 20/80 et 50/50 et une masse molaire inférieure ou égale à 1500 g.mol⁻¹,
le mélange M1 comprenant au moins 25% en masse d'au moins un oligomère de formule (I) dont au moins un des groupes R1 ou R2 est un groupement R3C(=O)-,
la composition tensioactive comprenant, par rapport à la masse du mélange M1, moins de 10% en masse de composé de formule (II) : 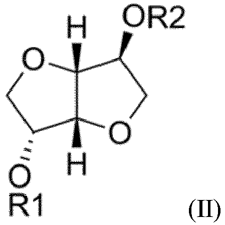 la composition tensioactive étant préparée selon au moins les étapes suivantes :
a) préparation d'une composition A comprenant un mélange d'oligoéthers par polyétherification de 1,3-propanediol (PDO) et d'isosorbide (ISO), le rapport molaire initial ISO/PDO étant compris entre 40/60 et 70/30
b) purification, par distillation moléculaire, de la composition A pour obtenir une composition B comprenant moins de 4% en masse d'isosorbide n'ayant pas été polyétherifié, par rapport à la masse de la composition B,
c) estérification, avantageusement par voie enzymatique, des oligoéthers de la composition B avec au moins un acide carboxylique de formule R3C(=O)OH, R3 étant une chaine carbonée comprenant entre 7 et 21 atomes de carbone, saturée ou insaturée, linéaire ou ramifiée.

2. Composition selon la revendication 1, ***caractérisée* en ce que** le mélange M1 présente un rapport molaire global ISO/PDO compris entre 20/80 et 50/50, ce rapport molaire correspondant au rapport entre toutes les unités ISO et toutes les unités PDO de tous les oligomères de formule (I) du mélange M1.

3. Composition selon l'une des revendications 1 à 2, ***caractérisée* en ce que** l'oligoéther -O-(ISO-PDO_{X})_{N}-ISO_{X'}-O- se termine par au moins un résidu de monomère d'isosorbide.

4. Composition selon l'une des revendications 1 à 3, ***caractérisée* en ce que** 10 à 100% des oligoéthers -O-(ISO-PDO_{X})_{N}-ISO_{X'}-O- des oligomères de formule (I) du mélange M1 présentent au moins un motif terminal qui est un résidu monomère d'isosorbide (ISO), avantageusement 70 à 100%.

5. Composition selon l'une des revendications 1 à 4, ***caractérisée* en ce qu'**elle comprend :
- 5 à 80 % d'oligomères R3C(=O)-O-(ISO-PDO_{X})_{N}-ISO_{X'}-OC(=O)R3, avantageusement 5 à 30 %,
- 20 à 70 % d'oligomères R3C(=O)O-(ISO-PDO_{X})_{N}-ISO_{X'}OH, avantageusement 30 à 60 %,
- 0 à 40% d'oligomères HO-(ISO-PDO_{X})_{N}-ISO_{X'}-OH, avantageusement 0 à 30%,
les pourcentages étant exprimés en masse, par rapport à la masse de la composition tensioactive.

6. Composition selon l'une des revendications 1 à 5, ***caractérisée* en ce que** R3 est un groupe alkyl linéaire de formule CₙH₂ₙ₊₁, n étant un nombre entier de 7 à 21, avantageusement de 13 à 17.

7. Composition selon l'une des revendications 1 à 6, ***caractérisée* en ce qu'**elle comprend moins de 5% en masse de composé de formule (II).

8. Composition selon l'une des revendications 1 à 7, ***caractérisée* en ce que** l'étape de purification est réalisée par distillation moléculaire, à une température comprise entre 110°C et 150°C et à une pression comprise entre 5 Pa et 10 Pa.

9. Formulation cosmétique contenant la composition tensioactive selon l'une des revendications 1 à 8.

## Patentansprüche

1. Tensidzusammensetzung mit einer Mischung M1, bestehend aus Oligomeren mit der Formel (I):
R1-O-(ISO- PDOₓ)_{N}-ISO_{x'}-OR2 (I)
- wobei -O-(ISO-PDO_{X})_{N}-ISO_{X}-OR2 für einen Oligoether steht, der mindestens einen Rest eines 1,3-propanediol (PDO)- Monomers und mindestens einen Rest eines Isosorbid- Monomers (ISO) enthält,
- R1 und R2 sind dabei ein Wasserstoffatom oder eine R3C(=0)- - Gruppe,
- R1 und R2 sind identisch oder unterschiedlich,
- R3 ist dabei eine Kohlenstoffkette, die zwischen 7 und 21 Kohlenstoffatome enthält, gesättigt oder ungesättigt, linear oder verzweigt,
- x ist eine Durchschnittszahl zwischen 1 und 5,
- x' ist eine Durchschnittszahl zwischen 0 und 1,
- N ist eine Durchschnittszahl zwischen 1 und 5,
jedes Oligomer mit der Formel (I) weist ein molares Verhältnis ISO/PDO zwischen 20/80 und 50/50 und eine molare Masse von kleiner oder gleich 1500 g.mol⁻¹,
die Mischung M1 weist dabei mindestens 25 Masse-% mindestens eines Oligomers der Formel (I) auf, bei dem mindestens eine der R1 oder R2- Gruppen eine R3C(=O)- - Gruppe ist, die Tensidzusammensetzung enthält, bezogen auf die Masse der Mischung M1 mindestens 10 Masse-% Verbindungen mit der Formel (II) 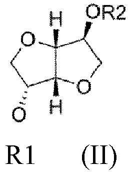 die Tensidverbindung wird entsprechend mindestens einem der nachfolgenden Schritte hergestellt:
a) Herstellung einer Zusammensetzung A mit einer Mischung aus Oligoether durch Polyveretherung von 1,3-propandiol (PDO) und Isosorbid (ISO), das ursprüngliche molare Verhältnis ISO/PDO liegt dabei zwischen 40/60 und 70/30
b) Reinigung durch molekulare Destillation der Zusammensetzung A um eine Zusammensetzung B zu erhalten, die weniger als 4 Masse-% nicht verethertes Isosorbid enthält, bezogen auf die Masse der Zusammensetzung B,
c) Veresterung, vorteilhafterweise auf enzymatischem Weg, der Oligoether der Verbindung B mit mindestens einer Carboxylsäure mit der Formel R3C(=O)OH, wobei R3 eine kohlenstoffhaltige Kette mit zwischen 7 und 21 Kohlenstoffatomen ist, gesättigt oder ungesättigt, linear oder verzweigt.

2. Zusammensetzung nach Anspruch 1, ***dadurch gekennzeichnet, dass*** die Mischung M1 ein globales molares Verhältnis ISO/PDO zwischen 20/80 und 50/50 aufweist, dieses molare Verhältnis entspricht der Beziehung zwischen allen ISO-Einheiten und allen PDO- Einheiten aller Oligomere der Formel (I) der Mischung M1.

3. Zusammensetzung nach einem der Ansprüche 1 bis 2, ***dadurch gekennzeichnet, dass*** der Oligoether -O-(ISO-PDO_{X})_{N}-ISO_{X'}-O- mit mindestens einem Isosorbid-Monomer- Rest endet.

4. Zusammensetzung nach einem der Ansprüche 1 bis 3, ***dadurch gekennzeichnet, dass*** 10 bis 100% der Oligoether -O-(ISO-PDO_{X})_{N}-ISO_{X'}-O- der Oligomere der Formel (I) der Mischung M1 mindestens ein Endmotiv aufweisen, bei dem es sich um einen Isosorbid- Monomer- Rest (ISO) handelt, vorteilhafterweise 70 bis 100%.

5. Zusammensetzung nach einem der Ansprüche 1 bis 4, **dadurch gekennzeichnet, dass** sie enthält:
- 5 bis 80 % Oligomere R3C(=O)-O-(ISO-PDO_{X})_{N}-ISO_{X'}-OC(=O)R3, vorteilhafterweise 5 bis 30 %
- 20 bis 70 % Oligomere R3C(=O)O-(ISO-PDO_{X})_{N}-ISO_{X'}OH, vorteilhafterweise 30 bis 60 %,
- 0 bis 40° 0 Oligomere HO-(ISO-PDO_{X})_{N}-ISO_{X'}-OH, vorteilhafterweise 0 bis 30%, die Prozentsätze werden in Masse angegeben, bezogen auf die Masse der Tensidzusammensetzung.

6. Verbindung nach einem der Ansprüche 1 bis 5, ***dadurch gekennzeichnet, dass*** es sich bei R3 um eine lineare Alkylgruppe der Formel CₙH2ₙ₊₁ handelt, wobei n eine ganze Zahl von 7 bis 21 ist, vorteilhafterweise von 13 bis 17.

7. Zusammensetzung nach einem der Ansprüche 1 bis 6, ***dadurch gekennzeichnet, dass*** sie mindestens 5 Masse-% der Verbindung der Formel (II) enthält.

8. Zusammensetzung nach einem der Ansprüche 1 bis 7, ***dadurch gekennzeichnet, dass*** der Reinigungsschritt durch molare Destillation ausgeführt wird, bei einer Temperatur zwischen 110° C und 150° C und einem Druck zwischen 5 Pa und 10 Pa.

9. Kosmetische Rezeptur, die die Tensidverbindung nach einem der Ansprüche 1 bis 8 enthält.

## Claims

1. A surfactant composition comprising a mixture M1 constituted by oligomers with formula (I) :
R1-O-(ISO-PDO_{X})_{N}-ISO_{X'}-OR2 (I)
- in which -O-(ISO-PDO_{X})_{N}-ISO_{X'}-O- represents an oligoether comprising at least one residue of a 1,3-propanediol (PDO) monomer and at least one residue of an isosorbide (ISO) monomer,
- R1 and R2 being a hydrogen atom or a R3C(=O)- group,
- R1 and R2 being identical or different,
- R3 being a carbon chain containing between 7 and 21 carbon atoms, which may be saturated or unsaturated, linear or branched,
- x being an average number in the range between 1 and 5,
- x' being an average number in the range between 0 and 1,
- N being an average number between 1 and 5,
each oligomer with formula (I) having an ISO/PDO molar ratio in the range 20/80 to 50/50 and a molar mass of less than or equal to 1500 g.mol⁻¹,
the mixture M1 comprising at least 25% by weight of at least one oligomer with formula (I) for which at least one of the groups R1 or R2 is a group R3C(=O)-,
the surface composition comprising, with respect to the mass of the mixture M1, less than 10% by weight of a compound with formula (II): 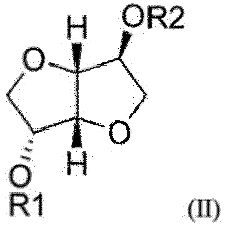 the surfactant composition being prepared in accordance with at least one of the following steps:
a) preparation of a composition A comprising a mixture of oligoethers by polyetherification of 1,3-propanediol (PDO) and isosorbide (ISO), the initial ISO/PDO molar ratio being in the range 40/60 to 70/30
b) purification of the composition A by molecular distillation in order to obtain a composition B comprising less than 4% by weight of isosorbide which has not been polyetherified, with respect to the mass of the composition B,
c) esterification, advantageously by an enzymatic pathway, of the oligoethers of the composition B with at least one carboxylic acid with formula R3C(=O)OH, R3 being a carbon chain containing between 7 and 21 carbon atoms which may be saturated or unsaturated, linear or branched.

2. The composition as claimed in claim 1, ***characterized* in that** the mixture M1 has an overall ISO/PDO molar ratio in the range 20/80 to 50/50, this molar ratio corresponding to the ratio between all of the ISO units and all of the PDO units of all of the oligomers with formula (I) of the mixture M1.

3. The composition as claimed in claim 1 or claim 2, ***characterized* in that** the oligoether -O-(ISO-PDO_{X})_{N}-ISO_{X'}-O- is terminated by at least one isosorbide monomer residue.

4. The composition as claimed in one of claims 1 to 3, ***characterized* in that** 10% to 100% of the oligoethers -O-(ISO-PDO_{X})_{N}-ISO_{X'}-O- of the oligomers with formula (I) of the mixture M1 have at least one terminal motif which is an isosorbide (ISO) monomer residue, advantageously 70% to 100%.

5. The composition as claimed in one of claims 1 to 4, ***characterized* in that** it comprises:
- 5% to 80% of the oligomers R3C(=O)-O-(ISO-PDO_{X})_{N}-ISO_{X'}-OC(=O)R3, advantageously 5% to 30%,
- 20% to 70% of the oligomers R3C(=O)O-(ISO-PDO_{X})_{N}-ISO_{X'}OH, advantageously 30% to 60%,
- 0 to 40% of the oligomers HO-(ISO-PDO_{X})_{N}-ISO_{X'}-OH, advantageously 0 to 30%,
the percentages being expressed by weight with respect to the mass of the surfactant composition.

6. The composition as claimed in one of claims 1 to 5, ***characterized* in that** R3 is a linear alkyl group with formula CₙH₂ₙ+1, n being a whole number from 7 to 21, advantageously from 13 to 17.

7. The composition as claimed in one of claims 1 to 6, ***characterized* in that** it comprises less than 5% by weight of the compound with formula (II).

8. The composition as claimed in one of claims 1 to 7, ***characterized* in that** the purification step is carried out by molecular distillation at a temperature in the range 110°C to 150°C and at a pressure in the range 5 Pa to 10 Pa.

9. A cosmetic formulation containing the surfactant composition as claimed in one of claims 1 to 8.
